# EUROPEAN PATENT SPECIFICATION

(11) **EP 2 854 759 B1**
(45) Date of publication and mention of the grant of the patent: **18.12.2019**
(21) Application number: 13723415.9
(22) Date of filing: 16.05.2013
(51) Int. Cl.: A61K 9/00, A61K 9/20, A61K 31/437, A61K 47/32, A61K 47/38, A61P 7/02

(54) **DOSAGE FORMS COMPRISING APIXABAN AND MATRIX FORMER**
DARREICHUNGSFORMEN MIT APIXABAN UND MATRIXFORMER
FORMES DE DOSAGE COMPRENANT APIXABAN ET UN AGENT DE FORMATION DE MATRICE

(30) Priority: 24.05.2012 EP 12004041; 24.05.2012 US 201213479549
(43) Date of publication of application: 08.04.2015
(73) Proprietor: ratiopharm GmbH, 89079 Ulm (DE)
(72) Inventor: MEERGANS, Dominique, 81379 München (DE); LEUTNER, Dirk, 80939 München (DE)
(74) Representative: Ter Meer Steinmeister & Partner
(86) International application number: PCT/EP2013/001461
(87) International publication number: WO 2013/174498

(56) References cited:
- WO-A1-03/026652
- WO-A1-2008/031782
- WO-A1-2010/147978
- WO-A2-2011/106478
- CN-A- 102 058 889
- VARMA M V S ET AL: "FACTORS AFFECTING MECHANISM AND KINETICS OF DRUG RELEASE FROM MATRIX-BASED ORAL CONTROLLED DRUG DELIVERY SYSTEMS", AMERICAN JOURNAL OF DRUG DELIVERY, ADIS INTERNATIONAL, AUCKLAND ; YARDLEY, PA, USA, vol. 2, no. 1, 1 January 2004 (2004-01-01) , pages 43-57, XP008043047, ISSN: 1175-9038, DOI: 10.2165/00137696-200402010-00003

## Description

The invention relates to oral dosage forms for modified release of apixaban. The invention also relates to methods of preparing said dosage forms and to an agglomerated mixture of matrix former and filler for preparing an oral dosage form for use in the treatment of venous thromboembolism.

Apixaban is a low molecular selective inhibitor of the enzyme factor Xa, participating in the blood coagulation system. Apixaban is classified as an antithrombotic drug which can be administered orally. Therefore, its possible medical indications are reported to be thrombosis treatment and thrombosis prophylaxis after orthopaedic operations as well as the prophylaxis of ischaemic apoplexia in case of atrial fibrillation, the prophylaxis of the acute coronary syndrome and the prophylaxis after thrombosis and pulmonary embolism.

The IUPAC name for apixaban is [INN] 1-(4-methoxyphenyl)-7-oxo-6-[4-(2-oxopiperidin-1-yl)phenyl]-4,5,6,7-tetrahydro-1H-pyrazolo[3,4-c]pyridine-3-carbamide. The chemical structure of apixaban is shown in the formula (1) below:

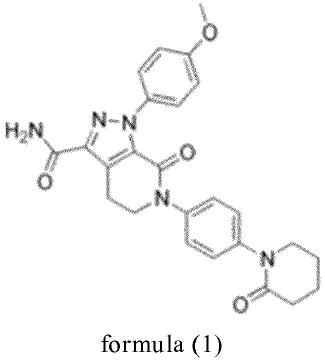

The synthesis of apixaban is described in WO 2003/026652 A1 by Bristol-Myers Squibb Company.

In WO 2006/078331 A2 and WO 2007/001385 A2 two polymorphous forms of apixaban are described. The H2-2 form is reported to be a needle-shaped dihydrate and further the H2-2 form is reported to be less stable than the granular, non-solvate N-1 form.

WO 2008/031782 A1 relates to a modified release pharmaceutical composition comprising plural mini-tablets, comprising a therapeutically effective amount of a Factor Xa inhibitor within a matrix of polymer(s). Apixaban is merely mentioned in a list of structurally and functionally diverse further Factor Xa inhibitors. D1 further discloses the particle size of granules comprised in the pharmaceutical composition wherein the granule contains active agent and further excipients, such as filler and polymer. The examples of D1 all refer to compound A, which is not apixaban but (E)-2-(5-chlorothien-2-yl)-N-{(3S)-1-[(s)-1-methyl-2-morpholin-4-yl-2-oxoethyl]-2-oxopyrrolidin-3-yl}ethenesulfonamide, which might be present in crystalline or non-crystalline form.

WO2011/106478 A2 relates to compositions comprising crystalline apixaban particles having a D90 equal to or less than 89 µm and a pharmaceutically acceptable carrier, wherein the compositions are used for the treatment and/or prophylaxis of thromboembolic disorders. The compositions according to D2 are immediate release compositions showing a high amount of dissolved/released agent after 60 minutes.

WO 03/026652 A1 relates to lactam-containing compounds and derivatives thereof, wherein these lactam-containing compounds are represented by the Formula "P₄-P-M-M₄" and wherein ring P, if present, is a 5-7 membered carbocycle or heterocycle and ring M is a 5-7 membered carbocycle or heterocycle. Compounds of the present invention are useful as inhibitors of trypsin-like serine proteases, specifically Factor Xa.

WO 2010/147978 A1 describes formulations comprising apixaban, reportedly providing a controlled release of the API. This controlled release is mainly provided as an osmotic-controlled release oral delivery system (OROS-formulation). Further, for comparison reasons, an immediate release formulation comprising crystalline apixaban prepared by dry granulation is disclosed. However, the production of OROS dosage forms is time consuming and needs a high expenditure of equipment, such as a laser for drilling the holes into the dosage form. Further, it is reported that dosage forms using the OROS may be responsible for the formation of benzoars or gastrointestinal obstruction in patients with known strictures. Furthermore, patients are often concerned by the excretion of apparently intact (OROS)-tablets in stools, such that they are not convinced that the tablet completely released the drug. This may lead to an insufficient patient compliance.

Further, it turned out that known oral pharmaceutical formulations comprising apixaban are still improvable with regard to bioavailability, stability and content uniformity.

Hence, it was an object of the present invention to provide an oral dosage of apixaban with modified release which overcomes the above-mentioned disadvantages.

In particular, it was an object of the present invention to provide the active agent in a form which possesses a superior dissolution profile and superior bioavailability as well as superior storage stability and content uniformity.

While developing apixaban formulations, the inventors of the present application were also confronted with the fact that crystalline apixaban can exist in different polymorphous forms which have different solubility profiles and may tend to change into different polymorphous forms. In a patient, the different solubility profile may lead to an undesirable, uneven rise in the concentration of the active agent. It was therefore an object of the present invention to provide an oral dosage form in which the release of apixaban is determined less by the solubility of the apixaban form used than by the dosage form in which the apixaban is contained. The aim was largely to avoid both inter-individual and also intra-individual deviations.

Finally, the objects should be achieved without the necessity of an apixaban solubility-enhancing pre-treatment.

### Summary of the invention

The above mentioned objects unexpectedly are achieved by an oral dosage form as described in claim 1. Hence, a subject of the present invention is an oral dosage form for modified release as defined in claim 1.

It was found that the dosage form of the present invention has a superior dissolution profile and superior permeability, resulting in an excellent bioavailability of the active pharmaceutical ingredient. Further, superior content uniformity was achieved even when direct-compression was applied. It was further found that the dosage form of the present invention was very stable over a prolonged period of time. The superior shelf-life properties are important, since apixaban is applied in low doses, and even little degradation of the drug can lower its beneficial effects significantly.

A further subject of the invention is a method of preparing an oral dosage form of the present invention comprising the steps of
i) mixing apixaban, matrix former and optionally further excipients
ii) optionally granulating the mixture of step i)
iii) processing the mixture of step i) or the granulates of step ii) and optionally further excipients into an oral dosage form
(iv) optionally film coating the dosage form.

Further, it has unexpectedly found that a specific mixture of matrix former and filler is particularly suitable for the preparation of oral dosage forms for the use in the treatment of venous thromboembolism. Hence, the use of an agglomerated mixture of matrix former and filler, wherein preferably the D10-value of the particle size distribution of the mixture is from 20 to 80 µm, the D50-value of the particle size distribution is from 70 to 160 µm and the D90-value is from 170 to 360 µm, for preparing an oral dosage form for use in the treatment of venous thromboembolism is described herein.

### Detailed description of the invention

The oral dosage form of the present invention is preferably a formulation for modified release (MR) of the active agent (apixaban).

The term "modified release" means, that after 60 minutes 1% to 20 %, preferably 3% to 15%, especially 4% to 8% of the active ingredient are released. The modified-release profile of the oral dosage form of the present invention is determined according to the USP method (paddle apparatus II, 900 ml test medium, phosphate buffer with 0.5% sodium dodecyl sulfate, pH 6.8, at 37°C and 75 rpm). Further, "modified release" means that 15% to 55%, preferably 19% to 44%, especially 27% to 36 % are released after 4 hours. Further, "modified release" means that 55% to 95%, preferably 60% to 89%, especially 65% to 80% are released after 10 hours.

In the context of this invention, the term "apixaban" comprises 1-(4-methoxyphenyl)-7-oxo-6-[4-(2-oxopiperidin-1-yl)phenyl]-4,5,6, 7-tetrahydro-1*H-*pyrazolo[3,4-c]pyridine-3-carbamide in accordance with formula (1) above. In addition, the term "apixaban" comprises all the pharmaceutically acceptable salts, hydrates and/or solvates thereof, for example apixaban dihydrate. Within the application, ratios or amounts of apixaban generally refer to the ratio or amount of apixaban as represented by formula I in unhydrated and unsolvated form.

In a particularly preferred embodiment the oral dosage form of the present invention comprises apixaban as the sole pharmaceutical active agent. In an alternative embodiment the pharmaceutical composition of the invention can comprise apixaban in combination with further pharmaceutical active agent(s).

Further, the apixaban contained in the present dosage form is used in particulate, crystalline form, wherein the particle size distribution has a D50-value of the apixaban particles from 11 to 75 µm, more preferably from 15 to 50 µm, particularly from 18 to 45 µm.

In a preferred embodiment the apixaban particle size distribution has a D90-value of 90 µm or greater, in particular of 95 to 500 µm. In another preferred embodiment the particle size distribution has a D90-value of the apixaban particles from 40 to 350 µm, more preferably from 50 to 120 µm, in particular from 55 to 100 µm.

In a preferred embodiment the apixaban particle size distribution has a D10-value of the apixaban particles from 0,1 to 80 µm, more preferably from 0.5 to 45 µm, in particular from 1.0 to 15 µm, especially from 1.1 to 9 um.

The particulate apixaban is in a crystalline form. The term "crystalline" can be used in the context of this invention to designate the state of solid substances in which the components (atoms, ions or molecules, i.e. in the case of apixaban, the apixaban molecules) are arranged in an orderly repeating pattern, extending in all three spatial dimensions and thus exhibit a periodic arrangement over a great range (= long-range order).

The particulate apixaban in the oral dosage form of the invention may consist of purely crystalline apixaban. Alternatively, it may also contain small amounts of non-crystalline apixaban components, provided that a defined melting point of crystalline apixaban can be detected in a DSC. A mixture containing 85 to 99.999% by weight crystalline apixaban and 0.001 to 15% by weight non-crystalline apixaban is preferred, more preferably 90 to 99.99% by weight crystalline apixaban and 0.01 to 10% non-crystalline apixaban, particularly preferably 95 to 99.9% by weight crystalline apixaban and 0.1 to 5% non- crystalline apixaban.

The D50-value of the particle size distribution can also be referred to as "average particle size". The particle distribution can be determined by means of laser diffractometry. In a preferred embodiment, a Malvern Instruments Mastersizer 2000 is used to determine the size, wet measurement with ultrasound 60 sec., 2,500 rpm, dispersed in sunflower oil, 10 % obscuration, the evaluation being performed according to the Mie-model). Alternatively, a Malvern Instruments Mastersizer 2000 can be used to determine the size, wet measurement with ultrasound 60 sec., 2,000 rpm, dispersed in liquid paraffin, the evaluation being performed according to the Fraunhofer-model).

The average particle size (D50-value), which is also denoted D50-value of the integral volume distribution, is defined in the context of this invention as the particle diameter at which 50 percent by volume of the particles have a smaller diameter than the diameter which corresponds to the D50-value. Likewise, 50 percent by volume of the particles have a larger diameter than the D50-value. Analogously, the D90-value of the integral volume distribution is defined as the particle diameter at which 90 percent by volume of the particles have a smaller diameter than the diameter which corresponds to the D90-value. Correspondingly, the D10-value of the integral volume distribution is defined as the particle diameter at which 10 percent by volume of the particles have a smaller diameter than the diameter which corresponds to the D10-value.

In a preferred embodiment of the present dosage form the particulate apixaban is present in the form of one of its polymorphs, preferably in the form of the N-1 polymorph or the H2-2 polymorph.

The term "N1" polymorph form refers to the polymorphous form as described in WO 2007/001385 as "N1", in particular form N1 being characterized by the two-theta angle positions in X-ray powder diffraction (XRPD):
10.0°±0.1°, 10.6°±0.1°, 12.3°±0.1°, 12.9°±0.1°, 18.5°±0.1°, 27.1°±0.1°

The term "H2-2" polymorph form refers to the polymorphous form as described in WO 2007/001385 as "H2-2", in particular form H2-2 being characterized by the two-theta angle positions in X-ray powder diffraction (XRPD): 5.8°±0.1°, 7.4°±0.1°, 16.0°±0.1°, 20.2°±0.1°, 23.5°±0.1°, 25.2°±0.1°

In a preferred embodiment of the oral dosage form apixaban is not present in a solubility-enhanced form. More preferably apixaban is not present in solubility-enhanced form such as a solid amorphous dispersion, nanoparticles, a nanosuspension, an apixaban/cyclodextrin complex, a softgel form, an amorphous form and/or a solution of apixaban in a liquid.

In a preferred embodiment in the apixaban particle size distribution the ratio of the D90-value to the D50-value is between 9 : 1 and 2:1, more preferably between 7 : 1 and 2.3 : 1, even more preferably between 6 : 1 and 2.7 : 1 and especially between 5 : 1 and 3 : 1. It appears that a small ratio of the D90-value to the D50-value seems to be favourable for good distribution of the active agent within the composition and therefore assures that the above-mentioned objects are achieved.

In a preferred embodiment the integral volume distribution of apixaban can be monomodal or bimodal, preferably monomodal. This means that the integral volume distribution of apixaban shows only one maximum.

In a preferred embodiment, the oral dosage form of the invention contains apixaban and matrix former, the weight ratio of apixaban to matrix former being 1 : 1 to 1 : 50, more preferably 1 : 2 to 1 : 30, even more preferably 1 : 3 to 1 : 20, especially 1 : 4 to 1 : 10.

The matrix former is preferably a substance for providing a scaffold (matrix) for embedding the active ingredient and to form a physical barrier which hinders the active ingredient from being released immediately from the dosage form. Thus, the matrix former may have the effect that the active ingredient can be released from the scaffold in continuous manner. Release of the drug from the matrix can be dissolution-controlled as well as diffusion-controlled mechanisms.

In a preferred embodiment of the present invention the matrix former can preferably be an organic substance such as wax, fat, oil, fatty acid, fatty alcohol, monoglyceride, diglyceride, triglyceride, and mixtures thereof. Wax, fat, oil, fatty acid, fatty alcohol, monoglyceride, diglyceride, triglyceride, and mixtures thereof are referred to as "fatty substances" within the present application.

Waxes are typically understood as meaning, phenomenologically, those substances which at 20°C (and 1013 hPa) are malleable, solid to brittle hard, have a coarsely to finely-crystalline structure, are translucent to opaque, but not glossy, melt at above 40°C (and 1013 hPa) without decomposition and have low viscosity even immediately above their melting point. Their consistency and solubility is highly temperature dependent, and they can be polished with light pressure. In the present context, waxes are preferably furthermore selected from the group consisting of, or comprising, esters of fatty acids, preferably higher fatty acids (C>12), with alcohols with the exception of glycerol, preferably long-chain aliphatic alcohols, in particular the above-mentioned wax alcohols (C>22), or mixtures of such esters. Waxes can be solid or liquid at room temperature and atmospheric pressure (25°C, 1013 hPa). Examples of waxes are natural waxes such as plant waxes, animal waxes or petrochemical waxes. Examples of these are carnauba wax, myrtle wax, Japan wax, sugarcane wax (of vegetable origin), beeswax (of animal origin), paraffin wax or microcrystalline wax (petrochemical waxes). Preferably not comprised by the expression wax are, in the present context, so-called synthetic waxes.

"Fats" are understood as meaning esters of glycerol (glycerine) with three fatty acids, that is to say a certain group of carboxylic acids. They are, therefore, a subgroup of the triglycerides. The fatty acids in the ester of a fat may be different (mixed glycerides) or, alternatively, identical. The fatty acids can be saturated or unsaturated and even-numbered or odd-numbered. The physical properties of a fat are determined by the chain lengths and in particular by the frequency of double bonds, i.e. the degree of saturation, in the fatty acids. Fats can be solid or liquid at room temperature and atmospheric pressure (25°C, 1013 hPa). Fats which are liquid at room temperature and atmospheric pressure (25°C, 1013 hPa) are also referred to as fatty oils. The animal fats contain predominantly mixed glycerides of three acids, which are palmitic acid, stearic acid and oleic acid. Apart from glycerides of palmitic acid, stearic acid and oleic acid, the vegetable oils predominantly contain glycerol esters of the polyunsaturated acids. Examples of vegetable oils are sesame seed oil, olive oil, almond oil, corn oil, palm oil, peanut oil, coconut oil, rapeseed oil, wheat germ oil, hemp seed oil, poppy seed oil, linseed oil, castor oil, sunflower oil, cotton seed oil and soybean oil. Presently, hydrogenated or hardened fats and oils are also comprised, for example hardened or hydrogenated castor oil (for example Cutina HR®), which is a preferred embodiment of the present matrix formers. Derivatized fats such as, for example, polyoxyethylated fats are less preferably present or advantageously not present at all.

Within the context of the present invention, oils comprise the fatty oils such as liquid paraffin and liquid propylene glycol diesters of fatty acids such as, for example, Miglyol® 840.

Fatty acids are saturated or unsaturated carboxylic acids. The fatty acids containing more than 12 carbon atoms, which can be employed in accordance with the invention, are referred to as higher fatty acids. As a rule, and preferred within the present context, fatty acids are unbranched, i.e. straight-chain. Examples of fatty acids which can be used in accordance with the invention are: tridecanoic acid, myristic acid, pentadecanoic acid, palmitic acid, margaric acid, stearic acid, nonadecanoic acid, arachinic acid, behenic acid, lignoceric acid, cerotic acid, melissic acid, palmitoleic acid, oleic acid, erucic acid, linoleic acid, linolenic acid, elaeostearic acid, arachidonic acid and clupanodonic acid. A preferred fatty acid is stearic acid.

Fatty alcohols are linear, saturated or unsaturated primary alcohols (1-alkanols) having at least six carbon atoms, preferably 12 to 32 carbon atoms. In most cases, they are obtained from fatty acids by means of reduction. In the present context, the fatty alcohols are also intended to include those which have more than 22 carbon atoms and which are sometimes referred to as "wax alcohols". Examples of fatty alcohols are: lauryl alcohol (1-dodecanol), 1-tridecanol, myristyl alcohol (1-tetradecanol), 1-pentadecanol, cetyl alcohol (1-hexadecanol), 1-heptadecanol, stearyl alcohol (1-octadecanol), oleyl alcohol (9-*cis*-octadecen-1-ol), erucyl alcohol (9-*trans*-octadecen-1-ol), ricinol alcohol (9-*cis*-octadecen-1,12-diol), linoleyl alcohol (*all*-cis-9,12-octadecadien-1-ol), linolenyl alcohol (*all-cis-*9,12,15-octadecatrien-1-ol), 1-nonadecanol, arachidyl alcohol (1-eicosanol), gadoleyl alcohol (9-*cis*-eicosen-1-ol), 5,8,1,14-eicosentetraen-1-ol, 1-heneicosanol, behenyl alcohol (1-docosanol), erucyl alcohol (1-3-*cis*-docosen-1-ol), brassidyl alcohol (1-3-*trans*-docosen-1-ol), lignoceryl alcohol, ceryl alcohol, myricyl alcohol. A preferred example is stearyl alcohol.

Examples of monoglycerides which can be employed in accordance with the invention are: glycerol (mono)behenate(2,3-dihydroxypropyl docosanate), glycerol monostearate, glycerol monocaprate, glycerol monococoate, glycerol monoerucate, glycerol monohydroxystearate, glycerol monoisostearate, glycerol monolanolate, glycerol monolaurate, glycerol monolinoleate, glycerol monomyristate, glycerol monooleate, glycerol monopalmitate, glycerol monoricinoleate, glycerol (mono)myristate, glycerol (mono)montanate and mixtures of these, such as, for example, glycerol palmitate stearate, the monoester of glycerol with a mixture of palmitic and stearic acid.

Examples of diglycerides which can be employed according to the invention are diesters with identical carboxylic acid radicals as those which have been mentioned for the monoglycerides, for example glycerol dilaurate, glycerol dimyristate, glycerol dioleate, glycerol dipalmitate, glycerol distearate, glycerol diisostearate, and mixtures of these, and diesters with different carboxylic acid radicals (mixed diesters) such as, for example, glycerol palmitostearate (Precirol®) and mixtures of these.

Triglycerides are esters of the trihydric alcohol glycerol (glycerine, propane-1,2,3-triol) with three carboxylic acid radicals (triester, also referred to as acyl glycerine). The carboxylic acid radicals can be identical or different. Diglycerides are esters of glycerol with two carboxylic acid radicals, i.e. only two of the three hydroxyl groups of the glycerol are esterified (diester). Depending on the position of the carboxylic acid radicals, one distinguishes between 1,2 and 1,3-diglycerides. Here too, the carboxylic acid radicals may be identical or different. Monoglycerides are esters of glycerol with only one carboxylic acid radical, i.e. only one of the three hydroxyl groups of the glycerol is esterified (monoester).

Matrix formers which are preferably employed are those which are solid at room temperature and atmospheric pressure (25°C, 1013 hPa).

In a preferred embodiment of the present invention the matrix former is a polymer, preferably a non-erodible polymer, wherein the polymer can preferably have a weight-average molecular weight ranging from 30.000 to 3,000,000 g/mol, preferably from more than 50,000 to 2,500,000 g/mol, more preferably from more than 125,000 to 2,200,000 g/mol, still more preferably from 250,000 to 2,000,000 g/mol, particularly preferred from 400,000 to 1,500,000 g/mol. The weight-average molecular weight is preferably determined in the context of this application by means of gel permeation chromatography.

Furthermore, a 2 % w/w solution of the non-erodible polymer in water at pH 7.0 preferably can have a viscosity of more than 2 mPas, more preferably of more than 5 mPas, particularly more than 8 mPas and up to 850 mPas when measured at 25°C. The viscosity can be determined according to Ph. Eur. 6.0, chapter 2.2.10. In the above definition the term "solution" may also refer to a partial solution (in case that the polymer does not dissolve completely in the solution).

Suitable non-erodible polymers, are e.g. cellulose derivatives, such as cellulose ether and cellulose ester, starch, corn starch, gum, shellac, synthetic polymers such as polyvinylchloride, polyvinyl alcohol or derivatives thereof, polyvinyl acetate, polyvinyl acetate/ polyvinylpyrollidone (Kollidon), polymers based on acrylic acid and/or methacrylic acid and their derivatives, a ionic exchange resins like polacrilin potassium, and mixture thereof. Mixtures of the above-mentioned substances and polymers can also be used.

In a preferred embodiment the matrix former is generally a substance specified in the European Pharmacopoeia which exhibits a water-solubility of less than 25 g/1, measured at 25° C. The matrix former preferably exhibits a solubility of 10 g/1 or less, more preferably 5 g/l or less, especially 0.001 to 2 g/l (determined using the column elution method in accordance with EU Directive 67/548 EEC, Annex V, Chap. A6).

Examples of matrix formers, preferably polymers, exhibiting the above specified water-solubility are cellulose ethers, such as methyl cellulose, ethyl cellulose, hydroxypropyl methyl cellulose or hydroxy propyl cellulose, cellulose esters, such as hydroxy propyl methyl cellulose acetate succinate (HPMC-AS) or hydroxy propyl cellulose phthalat, polyvinyl acetate/poly-vinylpyrrolidone (Kollidon), and non water-soluble polymers based on acrylic acid and/or methacrylic acid and their derivatives.

In a preferred embodiment the water solubility of the matrix former, preferably a polymer, can be pH-independent.

The following kinds of matrix formers, preferably polymers, exhibiting a pH-independent water-solubility are particularly preferred.
1. Polyvinyl acetate, polyvinyl acetate/polyvinylpyrrolidone copolymer, or polyvinyl acetate/polyvinylpyrrolidone mixture, and mixtures thereof.
   In particular, polyvinyl acetate copolymers such as copolymers comprising vinyl acetate and vinyl pyrrolidone units, having the structure 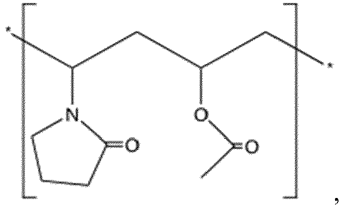 and polyvinyl acetate/polyvinylpyrrolidone mixtures are preferred. Particularly preferred are the polyvinyl acetate/polyvinylpyrrolidone mixtures containing 80% polyvinyl acetate and 19% of polyvinylpyrrolidone having a weight average molecular weight ranging from 44000-54000 g/mol (Kollidon® 30). These specific polyvinyl acetate/polyvinylpyrrolidone mixtures can be referred as Kollidon® SR.
2. Copolymers of methacrylic acid or methacrylic acid esters, preferably ethylacrylate methyl methacrylate and methacrylic acid methyl methacrylate. Particularly preferred is ethylacrylate methyl methacrylate trimethyl ammonio ethyl methacrylate chloride, for example Eudragit® RL PO (Röhm) and Eudragit® RS PO (Evonik Rohm).
   Preferred acrylic polymers are, for example, polyacrylate, polymethacrylate as well as derivatives and mixtures or copolymers thereof. The polyacrylates used in the invention preferably show the above-indicated parameters (e.g. weight average molecular weight, solubility, etc.).
   In a preferred embodiment the matrix former is (b) an acrylic polymer consisting of structures according to the general formulae (2) and (3). 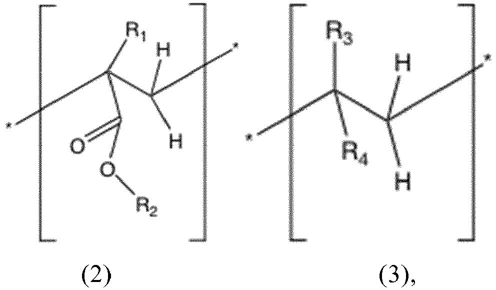 wherein in formulae (2) and (3)
   R₁ is a hydrogen atom or an alkyl group, preferably a hydrogen atom or a methyl group or an ethyl group, particularly preferred a methyl group;
   R₂ is a an alkyl group, preferably a hydrogen atom or a C₁-C₄ alkyl group, particularly preferred a methyl group, ethyl group or butyl;
   R₃ is a hydrogen atom or an alkyl group, preferably a hydrogen atom or a methyl group;
   R₄ is an organic group, preferably a carboxylic acid derivative, particularly preferred a group according to the formula -COOR₅,
   R₅ is an alkyl group or a substituted alkyl group, preferably a methyl, ethyl, propyl or butyl group, or -CH₂-CH₂-N(CH₃)₂ or -CH₂-CH₂-N(CH₃)₃⁺ halogen- (in particular Cl⁻) as substituted alkyl group.
   The acrylic polymer (b) according to formulae (2) and (3) is usually comprised of structures with a molar ratio of 1 : 40 to 40 : 1. The preferred ratio of the structures of formula (2) to structures of formula (3) is 2 : 1 to 1 : 1, particularly 1:1. When R₄ is -COO-CH₂-CH₂-N(CH₃)₃+Cl⁻, the ratio of structures according to formula (2) to structures of formula (3) preferably is 20 : 1 to 40 : 1.
   In case of an alternating copolymerization with a ratio of 1 : 1, this results in a preferred polymer according to formula (2+3) 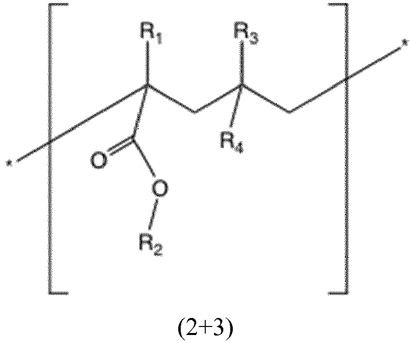
   Polyacrylates according to the formula (2+3) as mentioned above are particularly preferred, wherein R₁ and R₃ are alkyl, particularly methyl, R₂ is methyl and/or ethyl and R₄ is hydrogen or -COO-CH₂-CH₂-N(CH₃)₃+Cl⁻. A particularly preferred ratio of the structures according to formula (2) to the structures according to formula (3) is 1 : 1 or 1 : 20. A corresponding polymer preferably has a weight average molecular weight of 20,000 to 250,000 g/mol, more preferred of from 30,000 to 180,000 g/mol.
   In a particularly preferred embodiment in formula (2) (or in formula (2+3) as well), as indicated above, R₂ is both a methyl and a butyl group, whereby the ratio methyl to butyl group preferably is 1 : 1.
   Especially preferred are acrylic polymers being ternary polymers comprising the structures according to the general formulae (2a), (2b) and (3) 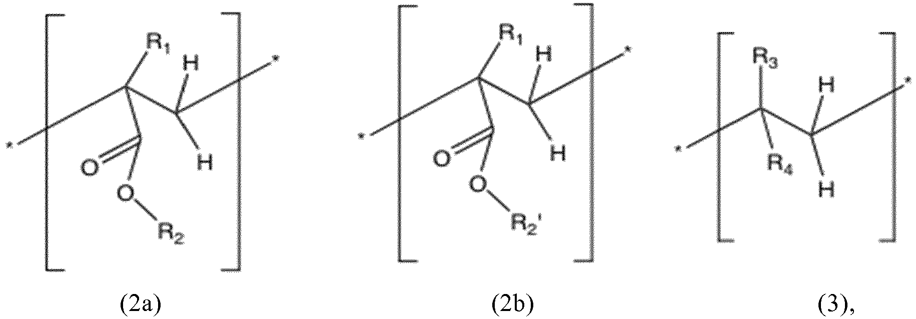 wherein R₁ and R₃ are hydrogen or alkyl, particularly methyl, R₂ is methyl, R_{2'} is ethyl and R₄ is -COO-CH₂-CH₂-N(CH₃)₃+Cl⁻, wherein the ratio of methyl methacrylate to ethyl acrylate to trimethylammonium ethyl methacrylate is 2 : 1 : 0.2 or 2 :1: 0.1.
3. Cellulose ethers, such as ethyl cellulose and hydroxypropyl cellulose, preferably ethyl cellulose having an average molecular weight of 150,000 to 300,000 g/mol and/or an average degree of substitution, ranging from 1.8 to 3.0, preferably from 2.2 to 2.6, more preferably hydroxypropyl cellulose, even more preferably a blend of lactose and hydroxyproyl methyl cellulose (hypromellose), especially preferably a spray agglomerated blend of 45-55 parts lactose monohydrate and 55-45 parts hypromellose (referred to as RetaLac®). Preferably, within said blend the hydroxypropyl methyl cellulose functions as matrix former and the lactose functions as filler.

In an alternative preferred embodiment the water-solubility of the matrix former, preferably a polymer, can be pH-dependent. In this case, the matrix former can preferably be a cellulose ester, such as hydroxypropyl cellulose acetate succinate, hydroxypropyl cellulose phthalat or carboxymethyl ethyl cellulose, or a polymer based on acrylic and/or methacrylic acid, preferably a polymer based on acrylic acid. This polymer based on acrylic acid is preferably a polymer of acrylic acid, which is cross-linked with polyalkenyl ethers, such as allyl pentaerytritol, and/or divinylglycol.
In a further preferred embodiment of the present invention, the matrix former is a swellable matrix former. The swellable matrix former is preferably a swellable polymer or a swellable substance with polymer-like properties. The swellable matrix former preferably has a swelling index of 1.2 to 6.0, preferably 1.5 to 4.5, more preferably 2.0 to 4.0. The swelling index indicates the volume in millilitres that 1 g substance, including any mucilage that may be adhering to it, occupies after swelling in an aqueous solution for 4 hours. The swelling index is determined in accordance with Ph.Eur., 6.1, Chapter 2.8.4.

Generally, the matrix former is a substance being capable of providing the desired modified release profile as described above. Hence, for example microcrystalline cellulose is not a matrix former according to the present invention, since it does not provide the desired modified release. Preferably, lactose, especially anhydrous lactose, microcrystalline cellulose, croscarmellose sodium, magnesium stearate, sodium lauryl sulfate and/or polyvinylpyrrolidon (in particular polyvinylpyrrolidon monomers) are not regarded as matrix formers within the present invention.

In a preferred embodiment of the present invention the oral dosage form can (in addition to the matrix former) preferably comprise one or more pharmaceutical excipient(s). The pharmaceutical excipients are excipients with which the person skilled in the art is familiar, such as those which are described in the European Pharmacopoeia (Ph.Eur.) and/or in the US Pharmacopoeia (USP).

Examples of the pharmaceutical excipients are filler (c1), glidant (c2), lubricant (c3), pore-forming substance (c4) and disintegrant (c5).

Generally, fillers (c1) are used to top up the volume for an appropriate orally deliverable dose, when low concentrations of the active pharmaceutical ingredients (about 30 wt.% or lower) are present. Examples of preferred fillers of the invention are calcium phosphate, calcium hydrogen phosphate, saccharose, calcium carbonate, calcium silicate, magnesium carbonate, magnesium oxide, starch, lactose, sucrose, glucose, mannitol, maltodextrin, glucopyranosyl mannitol, calcium sulfate, dextrate, dextrin, dextrose, hydrogenated vegetable oil and/or cellulose derivatives, such as microcrystalline cellulose and mixtures thereof. Preferred fillers are calcium hydrogen phosphate, microcrystalline cellulose and lactose, particularly preferred is lactose.

The fillers (c1) can be present in the oral dosage form of the present invention in an amount of 0 to 75 wt.%, preferably 5 to 70 wt.%, more preferably 10 to 65 wt.% and still more preferably 20 to 55 wt.% of the total weight of the oral dosage form.

Glidants (c2) can be used to improve the flowability. For example, talc can be used as glidant. More preferably, colloidal silica (for example AEROSIL®) is used. Preferably, the glidant can be present in an amount 0 to 3 wt.%, in particular, 0.1 to 2 wt.%, based on the oral dosage form. Preferably, the silica has a specific surface area of 50 to 400 m²/g, measured by gas adsorption according to Ph. Eur., 6.0, Chapter 2.9.26.

Lubricants (c3) are generally used in order to reduce sliding friction. In particular, the intention is to reduce the sliding friction found during tablet pressing between the punch moving up and down in the die and the die wall on the one hand and between the edge of the tablet and the die wall on the other hand. Suitable lubricants are, for example, stearic acid, adipic acid, sodium stearyl fumarate and/or magnesium stearate. Magnesium stearate is particularly preferred.

Lubricants (c3) are generally used in an amount 0 to 3 % by weight, preferably 0.1 to 2 wt.%, based on the total weight of the dosage form.

Pore-forming substance (c4) or "channeling agent" is in the art often synonymously used for the optional pore-forming material of the present invention. Since the pore-forming material is generally soluble in the gastrointestinal tract and leaches out from the oral dosage form, the pore-forming material can be described has having the effect of forming channels or pores, such as small holes within the tablet, through which the active ingredient can be released from the tablet matrix in a controlled manner. Thus, release of the active ingredient generally depends on dissolving the pore-forming material and thereby forming a porous matrix of capillaries such that the drug can leach out of the matrix.

The pore-forming substance (c4) usually has a water solubility of more than 50 mg/l, preferably more than 100 mg/l, at a temperature of 25°C and pH 5.0, more preferred of more than 250 mg/l and particularly preferred of more than 25 g/l. The water solubility of the pore-forming substance may range up to 500 g/l, or even up to 2.5 kg/l. The water-solubility is determined according to the column elution method of the Dangerous Substances Directive 67/548 EEC, Annex V, Chapter A6.

The pore-forming substance (c4) can be selected from inorganic substances, preferably from inorganic salts such as NaCl, KCl, Na₂SO₄. Furthermore, the pore-forming substance (c4) can be selected from organic substances, in particular from organic substances being solid at 30°C and having the above-mentioned water solubility. Suitable examples are PEG, particularly PEG having a weight average molecular weight of from 2,000 to 10,000 g/mol.

Furthermore, polyvinylpyrrolidone, preferably having a weight average molecular weight of from 5,000 to 29,000 g/mol, PEG with a weight average molecular weight of 380 - 4800, polyethylene oxide with a weight average molecular weight of less than 100,000 and a viscosity of less than 20 mPa●s, sugar alcohols like mannitol, sorbitol, xylitol, isomalt are also suitable as pore-forming substances.

The pore-forming material is usually contained in the tablet in an amount of 0 to 30 wt.%, preferably from 0.5 to 20 wt.%, most preferably from 2 to 15 wt.%, based upon the total weight of the oral dosage form.

Disintegrants (c5) are reported to be substances which accelerate the disintegration of a dosage form, especially a tablet, after having been placed in water. Suitable disintegrants are, for example, organic disintegrants, such as carrageenan, croscarmellose sodium, sodium carboxymethyl starch and crospovidone. crospovidone and/or croscarmellose sodium are particularly preferred.

Alternatively, inorganic alkaline disintegrants are used, preferably salts of alkali metals and alkaline metals. Preferred alkali and alkaline metals are sodium, potassium, magnesium and calcium. As anions, carbonate, hydrogen carbonate, phosphate, hydrogen phosphate and dihydrogen phosphate are preferred. The term "alkaline disintegrants" means disintegrants which, when dissolved in water, produce a pH level of more than 7.0. Examples for inorganic alkaline disintegrants are sodium hydrogen carbonate, sodium hydrogen phosphate and calcium hydrogen carbonate.

Disintegrants can be present in an amount of 0 to 20 wt.%, preferably 1 to 15 wt.%, more preferably 2 to 10 wt.% and still more preferably 3 to 8 wt.% based on the total weight of the dosage form.

In a preferred embodiment of the invention, the oral dosage form does not contain a disintegrant.

It lies in the nature of pharmaceutical excipients that they sometimes perform more than one function in a pharmaceutical formulation. In the context of this invention, in order to provide an unambiguous delimitation, the fiction will therefore preferably apply that a substance which is used as a particular excipient is not simultaneously also used as a further pharmaceutical excipient. For example, microcrystalline cellulose - if used as a filler - is not additionally used as a disintegrant, (even though microcrystalline cellulose also exhibits a certain disintegrating effect).

It lies in the nature of pharmaceutical excipients that they sometimes can perform more than one function in a pharmaceutical formulation. Therefore, some pharmaceutically acceptable ingredients may function as pharmaceutical excipient (c) as well as matrix former (b), i.e. the fact that an ingredient is used e.g. as a filler, or a disintegrant does not mean that it cannot also be acting as a matrix former (b). For example, sodium croscarmellose in an oral dosage form may both act as a disintegrant and as a matrix former.

However, in the context of this invention, in order to provide an unambiguous delimitation, the fiction will therefore preferably apply that a substance, which is used as a particular excipient, is not simultaneously also used as a further pharmaceutical excipient. For example, microcrystalline cellulose - if used as a wicking agent (c2) - is not also used as for example a disintegrant (even though microcrystalline cellulose also exhibits a certain disintegrating effect).

It has been unexpectedly found that specific combinations of matrix former and fillers have beneficial effects and solve the above-mentioned problems.

In a preferred embodiment of the present oral dosage form the oral dosage form comprises a filler (c1), wherein the weight ratio of matrix former (b) and filler (c1) is from 5 : 1 to 1 : 5, preferably from 3.5 : 1 to 1 : 3.5, more preferably from 2.5 :1 to 1 : 2.5, particularly from 1.5 : 1 to 1: 1.5.

It was found that a brittle and/or a non-brittle substance can be used as filler (c1).

Pharmaceutical excipients can generally be classified with regard to the change in the shape of the particles under compression pressure (compaction): plastic excipients are characterised by plastic deformation, whereas when compressive force is exerted on brittle excipients, the particles tend to break into smaller particles. Brittle behaviour on the part of the excipients can be quantified by the increase in the surface area in a moulding. In the art, it is customary to classify the brittleness in terms of the "yield pressure". According to a possible classification, the values for the "yield pressure" here are low for plastic substances but high in case of friable substances on the other hand (Duberg, M., Nystrom, C., 1982, "Studies on direct compression of tablets VI. Evaluation of methods for the estimation of particle fragmentation during compaction.", Acta Pharm. Suec. 19, 421-436; Humbert-Droz P., Mordier D., Doelker E., "Methode rapide de determination du comportement a la compression pour des etudes de preformulation", Pharm. Acta Helv., 57, 136-143 (1982)). The "yield pressure" describes the pressure that has to be reached for the excipient to begin to flow plastically.

The "yield pressure" is preferably calculated using the reciprocal of the gradient of the Heckel plot, as described in York, P., Drug Dev. Ind. Pharm. 18, 677 (1992). The measurement in this case is preferably made at 25 °C and at a deformation rate of 0.1 mm/s.

In the context of the present invention, an excipient (especially the filler) is deemed a non-brittle excipient if it has a "yield pressure" of no more than 120 MPa, preferably no more than 100 MPa, particularly preferably 5 to 80 MPa. An excipient is usually described as a brittle excipient if it has a "yield pressure" of more than 80 MPa, preferably more than 100 MPa, particularly preferably more than 120 MPa, especially more than 150 MPa. Brittle excipients may exhibit a "yield pressure" of up to 300 MPa or up to 400 MPa or even up to 500 MPa.

In a preferred embodiment the filler (c1) can be a brittle compound. Examples of brittle fillers are calcium phosphate, calcium hydrogen phosphate or (anhydrous) lactose. Brittle fillers (c1) can preferably be used in case that the matrix former is a cellulose derivative, such as hydroxypropylcellulose.

In an alternative embodiment the filler can be a non-brittle compound. A particularly preferred example of a non-brittle filler is microcrystalline cellulose.

It was unexpectedly found that mixtures of brittle and non-brittle fillers are particularly beneficial. Mixtures of brittle and non-brittle filler can preferably mixtures comprising for example lactose, calcium hydrogen phosphate as brittle fillers and for example microcrystalline cellulose, mannitol and starch as non-brittle fillers.
In a preferred embodiment the weight ratio of brittle filler to non-brittle filler is from 5 : 1 to 1 : 3, preferably from 4 : 1 to 1 : 2, more preferably from 3.5 : 1 to 1 : 1.5, in particular from 3 : 1 to 1 : 1.

The oral dosage form of the invention can preferably comprise a matrix former and a filler being present in an agglomerated mixture. In this mixture, the D10-value of the particle size distribution of the mixture is preferably from 20 to 80 µm, the D50-value of the particle size distribution is preferably from 70 to 160 µm and the D90-value is preferably from 170 to 360 µm.

The advantageous effects of the mixture of matrix former and filler do not only occur for apixaban dosage forms, but in general for dosage forms for use in the treatment of venous thromboembolism. Hence, a further subject of the present invention is the use of an agglomerated mixture of matrix former and filler, wherein preferably the D10-value of the particles size distribution of the mixture is from 20 to 80 µm, the D50-value of the particle size distribution is from 70 to 160 µm and the D90-value is from 170 to 360 µm for preparing an oral dosage form for use in the treatment of venous thromboembolism. All explanations given above for matrix former and filler also apply for the present subject of the present invention.

In a preferred embodiment the oral dosage form of the present invention can preferably comprise the following amounts of components:
1 to 40 mg apixaban, preferably 2 to 30 mg apixaban, particularly 2,55 to 20 mg apixaban,
5 to 200 mg matrix former, preferably 10 to 150 mg matrix former, particularly 20 to 100 mg matrix former,
5 to 200 mg filler, preferably 10 to 150 mg filler, particularly 20 to 100 mg filler,
0.1 to 15 mg glidant, preferably 0.5 to 10 mg glidant, particularlyl to 5 mg glidant
0.1 to 15 mg lubricant, preferably 0.5 to 10 mg lubricant, particularly 1 to 5 mg lubricant,
0 to 35 mg pore-forming substance, preferably 1 to 25 mg pore-forming substance, particularly 2 to 18 mg pore-forming substance,
0 to 25 mg disintegrant, preferably 1 to 17 mg disintegrant, particularly 2 to 13 mg disintegrant.

In a preferred embodiment the oral dosage form of the present invention can preferably comprise:
1 to 20 wt. % apixaban, preferably 3 to 18 wt.% apixaban, particularly 4 to 13 wt.% apixaban,
5 to 80 wt. % matrix former, preferably 12 to 65 wt.% matrix former, particularly
20 to 55 wt.% matrix former,0 to 75 wt.% filler, preferably 10 to 65 wt.% filler, particularly 20 to 55 wt.% filler,
0 to 3 wt.% glidant, preferably 0.1 to 2.5 wt.% glidant, particularly 0.1 to 2 wt.% glidant,
0 to 3 wt.% lubricant, preferably 0.1 to 2.5 wt.% lubricant, particularly 0.1 to 2 wt.% lubricant,
0 to 30 wt.% pore-forming substance, preferably 0.5 to 20 wt.% pore-forming substance, particularly 2 to 15 wt.% pore-forming substance,
0 to 20 wt.% disintegant, preferably 1 to 15 wt.% disintegrant, particularly 2 to 10 disintegrant,
based on the total weight of the dosage form.

In a preferred embodiment the oral dosage form of the present invention is in the form of a capsule or a tablet, preferably a tablet, more preferably a tablet for peroral use.

Further, the oral dosage form, preferably the tablet, of the invention preferably has contents of active agent(s), which lie within the concentration of 90 to 110%, preferably 95 to 105%, especially preferred from 98 to 102% of the average content of the active agents(s). This "content uniformity" is determined with a test in accordance with Ph. Eur., 6.0, Chapter 2.9.6. According to that test, the content of the active agents of each individual tablet out of 20 tablets must lie between of 90 to 110%, preferably 95 to 105%, especially 98 to 102% of the average content of the active agents(s). Therefore, the content of the active drugs in each tablet of the invention differs from the average content of the active agent by at most 10%, preferably at most 5% and especially at most 2%.

In addition, the oral dosage form, preferably the resulting tablet, preferably has a friability of less than 5%, particularly preferably less than 2%, especially less than 1%. The friability is determined in accordance with Ph. Eur., 6.0, Chapter 2.9.7. The friability of tablets generally refers to tablets without coating.

The dosage form of the invention tablets may be a peroral tablet, which can be swallowed unchewed. The tablet can preferably be film coated.

Generally, film coatings which do not affect the release of the active agent(s) and film coatings affecting the release of the active agent(s) can be employed with tablets according to invention. The film coatings which do not affect the release of the active agent(s) are preferred.

Preferred examples of film coatings which do not affect the release of the active ingredient can be those including poly(meth)acrylate, methylcellulose (MC), hydroxypropyl methylcellulose (HPMC), hydroxypropyl cellulose (HPC), hydroxyethyl cellulose (HEC), polyvinylpyrrolidone (PVP) and mixtures thereof.

These polymers can have a weight-average molecular weight of 10,000 to 150,000 g/mol.

In an alternative preferred embodiment, the film coating can affect the release of the active agent. Examples for film coatings affecting the release of the active agent are gastric juice-resistant film coatings and retard coatings.

Further, the coating can be free from active ingredient. However, it is also possible that the coating can contain an active ingredient (apixaban). In such a case, this amount of active ingredient would function as an initial dose. In such a case, the coating preferably can comprise 1 to 45 wt.%, preferably 5 to 35 wt.%, more preferably 10 to 30 wt.% of apixaban, based on the total amount of apixaban contained in the tablet.

In the preferred case that the film coating does not contain an active agent (a) or (b), said coating can have a thickness of 2 µm to 100 µm, preferably from 20 to 60 µm. In case of a coating containing an active agent (a) or (b), the thickness of the coating is usually 10 µm to 200 µm, preferably from 50 to 125 µm.

The oral dosage form, preferably the tablets, of the invention preferably can have a hardness of 25 o to 250 N, particularly preferably of 30 to 180 N, more preferably 40 to 150 N. The hardness is determined in accordance with Ph.Eur., 6.0, Chapter 2.9.8.

In a preferred embodiment of the oral dosage form of the invention the dissolution profile of said dosage form shows a substantial zero-order kinetic in the range of 0 to 80% dissolution, preferably 0-60 % dissolution, more preferably 0-20% dissolution. The release of substantial zero order can be defined as a linear release profile, preferably with a deviation of at most +/- 15%. The substantial zero-order kinetic is essentially independent from the concentration of the apixaban in the oral dosage form. Consequently, it is suitable to take a linear approach on a time/concentration graph. The constant release of the active agent preferably provides for reliably constant, superior plasma levels of the active agent.

The oral dosage form of the invention can preferably be capable of releasing the pharmaceutical active agent at a substantial zero order rate for a period of between 1 and 24 hours, preferably for between 2 and 16 hours, more preferably for between 4 and 12 hours, most preferred for between 6 and 10 hours.

A further subject of the invention is a method for preparing the dosage form of the present invention comprising the steps of
i) mixing apixaban, matrix former and optionally further pharmaceutical excipients,
ii) optionally granulating the resulting mixture of step i),
iii) processing the mixture resulting from step i) or the granulates resulting from step ii) and optionally further excipient(s) into an oral dosage form, and
iv) optionally film-coating the dosage form.

"Mixing" is understood in the context of the present invention as meaning a process of combining substances with the aim of achieving a substantially homogeneous distribution of different substances by the action of mechanical forces. Mixing for the purposes of the invention is performed in conventional mixing devices, such as roll mixers, shaking mixers, free-fall mixers, shear mixers, ploughshare mixers, planetary mixing kneaders, Z or sigma kneaders or fluid or intensive mixers. A free-fall mixer is preferably used.

The time for the step of mixing i) may, for example, be 0.5 minutes to 1 hour, such as about 2 minutes to 50 minutes, or 5 minutes to 45 minutes.

The mixing step can be accompanied by a step of jointly comminuting the particle sizes of (a) apixaban, the matrix former and the optional further excipient(s), for example by grinding them jointly, wherein it is assured that the average particle size of apixaban is from 10 to 500 µm.

In a preferred embodiment, step i) can be characterized by mixing the apixaban with matrix former and one or more excipient(s). The mixing i) can be carried out with conventional mixing devices. In order to ensure an even distribution, mixing in intensive mixers is preferable. Suitable mixing devices can preferably be compulsory mixers or free fall mixer, for example a Turbula® T 10B (Bachofen AG, Switzerland). Mixing can be carried out, for example, for 1 minute to 1 hour, preferably for 5 to 30 minutes.

In an alternative embodiment, mixing i) can be conducted such that the apixaban can be mixed with a first part of the matrix former and optionally further excipient(s) in a mixing device, for example in a high shear or tumbler mixer. After this first mixing step a second part of matrix former and optionally further excipient(s) can be added, which may be followed by a second mixing step. This procedure can be repeated until the last part of the matrix former and optionally further excipient(s) is used, preferably one to five times. This kind of mixing can assure an even distribution of active agent and provide a mass for further processing in step (ii), for example for a tabletting process.

In step ii) the optional granulation of the mixture resulting from step i) can be carried out. The granulation may be dry granulation, wet granulation or melt-granulation, though wet and dry granulations are preferable. The two latter types of granulation have the advantage of being gentler for active agents and excipients. Furthermore, dry granulation in particular is an economical process.

"Granulating" is generally understood to mean the formation of relatively coarse or granular aggregate material as a powder by assembling and/or aggregating finer powder particles (agglomerate formation, or build-up granulation) and/or the formation of finer granules by breaking up coarser aggregates (disintegration, or break-down granulation).

Dry granulation is generally carried out using pressure or temperature. Wet granulation is generally carried out using dispersants and optionally surface stabilisers. Granulation is generally carried out in conventional granulating devices, such as extruder, perforated-disk, perforated-roll, or fluidised-bed granulators. Compulsory mixers or spray dryers can also be used.

The granulation time, especially in the case of wet granulation is usually 1 minute to 1 hour, preferably 2 minutes to 30 minutes. Dry granulation is usually carried out as a continuous process.

In one embodiment of the process of the invention, in which dry granulation is contemplated, the mixture is compacted into a slug of material. The compacting conditions here are preferably selected such that the compacted material has a density of 1.03 to 1.8 g/cm³, especially 1.05 to 1.7 g/cm³. The compacting is preferably carried out in a roll granulator. The rolling force per roll width in this case is preferably 2 to 50 kN/cm, more preferably 4 to 30 kN/cm, especially 10 to 25 kN/cm. The gap width of the roll granulator is, for example, 0.8 to 5 mm, preferably 1 to 4 mm, more preferably 1.5 to 3 mm, especially 1.8 to 2.8 mm. After that, the compacted material is preferably granulated. The granulating can generally be performed with processes known in the state of the art.

Wet granulation can also be performed with conventional methods. The substances from step i) are wetted with a granulation liquid or suspended in a granulation liquid. Suitable liquids for preparing the granulation liquid are, for example, water, alcohols and mixtures thereof. A mixture of water and ethanol is preferred. The wet granulation can, for example, be performed in a fluidized-bed granulator, such as Glatt® GPCG 3, or a mixer, such as a compulsory mixer. If wet granulation is performed, an additional drying step is usually employed. "Drying" for the purposes of this invention is understood to mean the separation of liquids adhering to solids. Drying generally takes place in conventional drying equipment, such as cabinet or tray dryers, vacuum dryers, fluidized-bed dryers, spray dryers or freeze dryers. The drying and granulation process is preferably performed in one and the same apparatus. The granules are then dried and optionally screened. A suitable granulating machine is, for example, Diosna® P1/6.

In a preferred embodiment, the granulation conditions are selected such that the resulting particles (granules) have an average particle size (D50) of 50 to 600 µm, more preferably 100 to 500 µm, even more preferably 150 to 400 µm, especially 200 to 350 µm.

In addition, the granulation conditions are preferably selected such that the resulting granulates have a bulk density of 0.2 to 0.85 g/ml, more preferably 0.3 to 0.8 g/ml, especially 0.4 to 0.7 g/ml. The Hausner factor is usually in the range of 1.03 to 1.3, more preferably of 1.04 to 1.20 and especially of 1.04 to 1.15. The "Hausner factor" in this context means the ratio of compacted density to bulk density.

In step iii) the mixture resulting from step i) or the granules resulting from step ii) and optionally further excipient(s) are processed into an oral dosage form. In a preferred embodiment step iii) can include compressing the mixture resulting from step i) or the granules resulting from step ii) and optionally further excipient(s).

The compression of the mixture of step i) can be preferably a direct compression. The compression can be performed with tabletting machines known in the prior art, such as eccentric presses or rotary presses. In the case of rotary presses, a compressive force of 1 to 50kN, preferably 2 to 40 kN, more preferably 2.5 to 35 kN, can preferably be applied. As an example, the Fette® 102i press (Fette GmbH, Germany) or a Riva® Piccola (Riva, Argentina) can be used. In the case of eccentric presses, a compressive force of 1 to 20 kN, preferably 2.5 to 10 kN, can be preferably applied. For example, the eccentric press Korsch® EK0 can be used.

In step iv) the tablet can preferably be film-coated, either with a film coating affecting the release of the active agent or with a film coating not affecting the release of the active agent. A film coating without affecting the release of the active agent is preferred.

In a preferred embodiment the dosage form may be administered in a single daily dose or in divided doses, two to six times a day. In certain embodiments of the invention the dosage form of apixaban may be administered less frequent then once daily, e.g. every second, third or fourth day. In particular, the dosage form of the present invention is administered twice daily. It has been unexpectedly found that the dosage form of the present invention can be administered independently from the meals of the patient, i.e. the dosage forms of the present invention are suitable to be administered before, during or after the meals.

### EXAMPLES

Apixaban used in the examples is prepared e.g. according to WO 2006/078331 A2 or WO 2007/001385 A2. If necessary, particle size is adjusted by means known in the art, e.g. milling and/or sieving.

### Example 1

To 5 g apixaban (N-1 polymorph, D90= 96.8 µm, D50= 41.3 µm; D10= 6.8 µm) and 44 g of hydroxypropyl methylcellulose/lactose (RetaLac®) 0.5 g of silicium dioxide (AEROSIL® 200) sieved through a 500 µm mesh was added. The resulting mixture was blended in a Turbula® T10B Mixer at 23 rpm for 25 minutes. After addition of 0.5 g magnesium stearate and blending for further 5 minutes the powdery blend was compressed on an eccentric press Korsch® EKO to 6 mm round, biconvex tablets (100 mg) with a hardness of 100 to 110N each containing

| | |
|---|---|
| apixaban | 10 mg |
| hydroxypropyl methylcellulose/lactose | 88 mg |
| silicium dioxide | 1 mg |
| magnesium stearate | 1 mg |

### Example 2

1.05 g apixaban (H2-2 polymorph, D90= 57.3 µm; D50= 11.1 µm; D10= 1.3 µm), 4 g polyvinyl acetate/polyvinylpyrrolidone (Kollidon SR), 3 g lactose (Tablettose® 80), 3 g calcium hydrogen phosphate (Dicafos AN) and 0.1 g silicium dioxide (AEROSIL® 200) were sieved through a 1000 µm mesh. The resulting mixture was blended in a Turbula® T10B Mixer at 23 rpm for 15 minutes. After addition of 0.2 g magnesium stearate sieved through a 500 µm mesh and blending for further 5 minutes the powdery blend was compressed on an eccentric press Korsch® EKO to 6 mm round, biconvex tablets with a hardness of 100 to 140 N each containing

| | |
|---|---|
| apixaban (calculated as free base without water of hydration) | 10 mg |
| polyvinylacetate/polyvinylpyrrolidone | 40 mg |
| lactose | 30 mg |
| calcium hydrogen phosphate | 30 mg |
| silicium dioxide | 1 mg |
| magnesium stearate | 2 mg |

### Example 3

To 5.25 g apixaban (H2-2 polymorph) and 43.75 g of hydroxypropyl methylcellulose/lactose (RetaLac®) 0.5 g of silicium dioxide (AEROSIL® 200), sieved through a 500 µm mesh, was added. The resulting mixture was blended in a Turbula® T10B Mixer at 23 rpm for 25 minutes. After addition of 0.5 g magnesium stearate and blending for further 5 minutes the powdery blend was compressed on an eccentric press Korsch® EKO to 6 mm round, biconvex tablets with a hardness of approx. 130 N each containing
apixaban (calculated without water of hydration)
hydroxypropylmethyl celluose/lactose
silicium dioxide
magnesium stearate

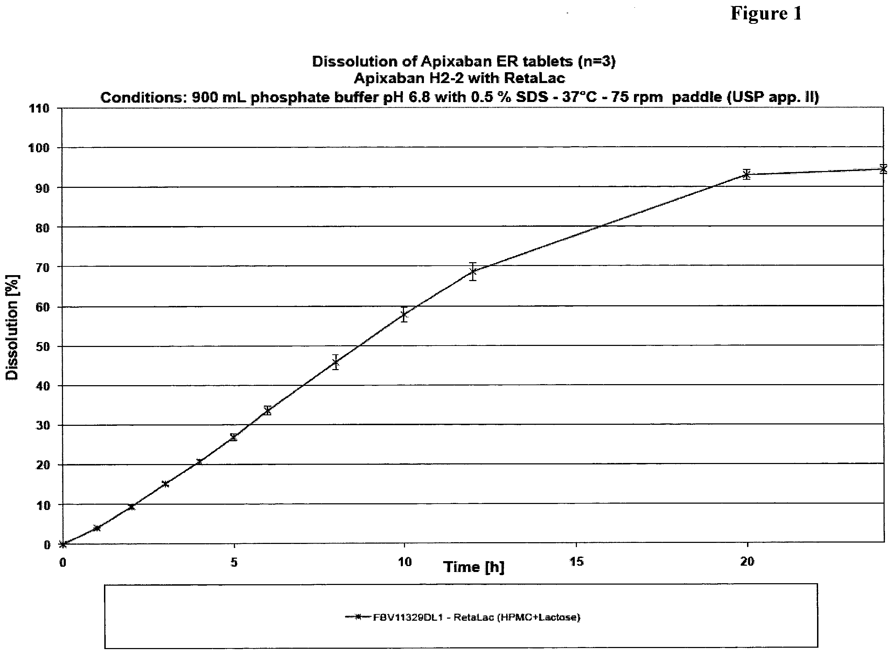

The dissolution profile of this dosage form is shown in Figure 1.

### Example 4

5 g apixaban (N-1 polymorph, D90= 57.3 µm; D50= 11.1 µm; D10= 1.3 µm) and 22.5 g ethyl acrylate/methyl methacrylate/methacrylic acid ester with quaternary ammonium groups copolymer (Eudragit® RL PO) and 12.5 g lactose (Granulac® 200) were blended in a Turbula® T10B Mixer at 23 rpm for 5 minutes. The resulting mixture was granulated with water and dried at 40°C. To the granulates sieved through a 1250 µm mesh 8.5 g microcrystalline cellulose (Microcel 102 SP) and 0,5 g silicium dioxide (AEROSIL® 200), both sieved through a 800 µm mesh, were added. The resulting mixture was blended in a Turbula® T10B Mixer at 23 rpm for 15 minutes. After addition of 1 g magnesium stearate and blending for further 5 minutes the blend was compressed on an eccentric press Korsch® EKO to 6 mm round, biconvex tablets with a hardness of 70 to 90 N each containing

| | |
|---|---|
| apixaban | 10 mg |
| acrylate/methcrylate based copolymer | 45 mg |
| lactose | 25 mg |
| microcrystalline cellulose | 17 mg |
| silicium dioxide | 1 mg |
| magnesium stearate | 2 mg |

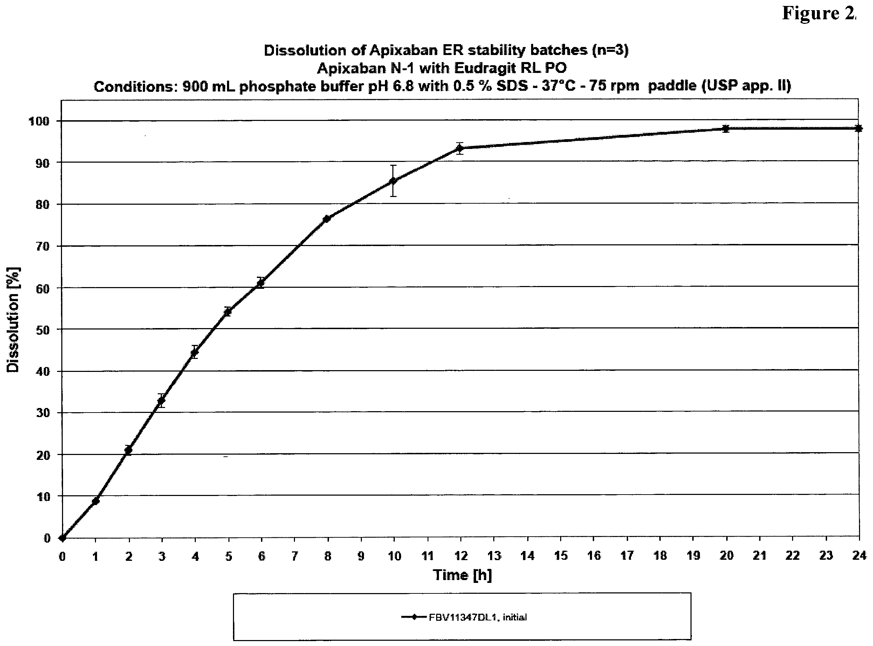

The dissolution profile of this dosage form is shown in Figure 2.

### Example 5

5.25 g apixaban (H2-2 polymorph, D90= 96.8 µm; D50= 41.3 µm; D10= 6.8 µm) and 22.5 g ethyl acrylate/methyl methacrylate/methacrylic acid ester with quaternary ammonium groups copolymer (Eudragit® RL PO) and 12.5 g lactose (Granulac® 200) were blended in a Turbula® T10B Mixer at 23 rpm for 5 minutes. The resulting mixture was granulated with water and dried at 40°C. To the granulates sieved through a 1250 µm mesh 8.5 g microcrystalline cellulose (Avicel PH 102) and 0.5 g silicium dioxide (AEROSIL® 200), both sieved through a 800 µm mesh, were added. The resulting mixture was blended in a Turbula® T10B Mixer at 23 rpm for 15 minutes. After addition of 1 g magnesium stearate and blending for further 5 minutes the blend was compressed on an eccentric press Korsch® EKO to 6 mm round, biconvex tablets with a hardness of 100 to 110 N each containing

| | |
|---|---|
| apixaban (calculated without water of hydration) | 10 mg |
| acrylate/methcrylate based copolymer | 45 mg |
| lactose | 25 mg |
| microcrystalline cellulose | 17 mg |
| silicium dioxide | 1 mg |
| magnesium stearate | 2 mg |

## Claims

1. Oral dosage form for modified release containing
a) particulate, crystalline apixaban and
b) matrix former,
wherein the apixaban particle size distribution has a D50-value of 11 to 75 µm, and wherein the matrix former is selected from cellulose ether, cellulose ester, starch, gum, shellac, fatty substances, polyvinylchloride, polyvinyl alcohol polyvinyl acetate or copolymers thereof, polymers based on acrylic acid and/or methacrylic acid and ion exchange resins, wherein the fatty substances refer to wax, fat, oil, fatty acid, fatty alcohol, monoglyceride, diglyceride, triglyceride and mixtures thereof, and
wherein modified release means that after 60 minutes 1% to 20% of the active agent are released, determined according to the USP method, paddle apparatus II, 900 ml test medium, phosphate buffer with 0.5% sodium dodecyl sulfate, pH 6.8 at 37°C and 75 rpm.

2. Oral dosage form according to clam 1, wherein the dosage form shows 1% to 20% release after 60 minutes, 15% to 55% after 4 hours and 55% to 95% after 10 hours, determined according to the USP method, paddle apparatus II, 900 ml test medium, phosphate buffer with 0.5% sodium dodecyl sulfate, pH 6.8 at 37°C and 75 rpm.

3. Oral dosage form according to claim 1 or 2, wherein the apixaban is present in form of the N1-polymorph or in form of the H2-2-polymorph,
wherein the N1-polymorph is **characterized by** the two-theta angle positions in X-ray powder diffraction (XRPD): 10.0°±0.1°, 10.6°±0.1°, 12.3°±0.1°, 12.9°±0.1°, 18.5°±0.1°, 27.1°±0.1, and
wherein the H2-2-polymorph is **characterized by** the two-theta angle positions in X-ray powder diffraction (XRPD): 5.8°±0.1°, 7.4°±0.1°, 16.0°±0.1°, 20.2°±0.1°, 23.5°±0.1°, 25.2°±0.1.

4. Oral dosage form according to any one of claim 1 to 3, wherein in the apixaban particle size distribution the ratio of D90-value to D50-value is between 9:1 to 2:1

5. The oral dosage form according to any one of claims 1 to 4, wherein the weight ratio of apixaban to matrix former is 1 : 1 to 1 : 50.

6. The oral dosage form according to any one of claims 1 to 5, wherein the matrix former is a non-erodible polymer, wherein the non-erodible polymers are cellulose derivatives, such as cellulose ether and cellulose ester, starch, corn starch, gum, shellac, synthetic polymers such as polyvinylchloride, polyvinyl alcohol or derivatives thereof, polyvinyl acetate, polyvinyl acetate/ polyvinylpyrollidone (Kollidon), polymers based on acrylic acid and/or methacrylic acid and their derivatives, a ionic exchange resins like polacrilin potassium, and mixture thereof.

7. The oral dosage form according to any one of claims 1 to 6, wherein the matrix former has a water solubility at 25 °C of less than 33 mg/l, determined by the column elution method in accordance with EU Directive 67/548 EEC, Annex V, Chap. A6.

8. The oral dosage form according to any one of claims 1 to 7, wherein the matrix former has a substance having a swelling ratio of 1.5 to 4.5, determined in accordance with Ph.Eur., 6.1, Chapter 2.8.4.

9. The oral dosage form according to any one of claims 1 to 8 further comprising a (c1) filler.

10. The oral dosage form according to claim 9, wherein the matrix former (b) and filler (c1) are present in an agglomerated mixture.

11. The oral dosage form according to any one of claims 1 to 10 comprising
2.5-20 mg apixaban
20-100 mg matrix former
20-100 mg filler
1-5 mg glidant, and
1-5 mg lubricant

12. The oral dosage form according to any one of claims 1 to 11 in form of a tablet having a content uniformity of 95 to 105%, a friability of less than 5% and/or a hardness of 30 to 180 N.

13. The oral dosage form according to any one of claims 1 to 12, wherein the dissolution profile of said dosage form shows a substantial zero-order kinetic in the range of 0 to 80% dissolution.

14. A method of preparing an oral dosage in accordance with any one of claims 1 to 13 comprising the steps of
i) mixing apixaban, matrix former and optionally further pharmaceutical excipient(s)
ii) optionally granulating the mixture of step i)
iii) processing the mixture of step i) or the granulates of step ii) and optionally further excipient(s) into an oral dosage form
iv) optionally film-coating the dosage form.

## Patentansprüche

1. Orale Darreichungsform zur modifizierten Freisetzung enthaltend
a) teilchenförmiges, kristallines Apixaban und
b) Matrixbildner,
wobei die Apixaban-Partikelgrößenverteilung einen D50-Wert von 11 bis 75 µm aufweist, und
wobei der Matrixbildner ausgewählt ist aus Celluloseether, Celluloseester, Stärke, Gummi, Schellack, Fettsubstanzen, Polyvinylchlorid, Polyvinylalkoholpolyvinylacetat oder Copolymeren davon, Polymeren auf Basis von Acrylsäure und/oder Methacrylsäure und Ionenaustauscherharzen, wobei sich die Fettsubstanzen auf Wachs, Fett, Öl, Fettsäure, Fettalkohol, Monoglycerid, Diglycerid, Triglycerid und Mischungen davon beziehen, und
wobei modifizierte Freisetzung bedeutet, dass nach 60 Minuten 1% bis 20% des Wirkstoffs freigesetzt werden, bestimmt nach dem USP-Verfahren, Paddelvorrichtung II, 900 ml Testmedium, Phosphatpuffer mit 0,5% Natriumdodecylsulfat, pH 6,8 bei 37°C und 75 U/min.

2. Orale Darreichungsform nach Anspruch 1, wobei die Darreichungsform 1% bis 20% Freisetzung nach 60 Minuten, 15% bis 55% nach 4 Stunden und 55% bis 95% nach 10 Stunden, bestimmt nach dem USP-Verfahren, Paddelvorrichtung II, 900 ml Testmedium, Phosphatpuffer mit 0,5% Natriumdodecylsulfat, pH 6,8 bei 37°C und 75 U/min zeigt.

3. Orale Darreichungsform nach Anspruch 1 oder 2, wobei das Apixaban in Form des N1-Polymorphs oder in Form des H2-2-Polymorphs vorliegt,
wobei der N1-Polymorph durch die Zwei-Theta-Winkelpositionen in der Röntgenpulverdiffraktion (XRPD) gekennzeichnet ist: 10,0°±0,1°, 10,6°±0,1°, 12,3°±0,1°, 12,9°±0,1°, 18,5°±0,1°, 27,1°±0,1 und
wobei der H2-2-Polymorph durch die Zwei-Theta-Winkelpositionen in der Röntgenpulverdiffraktion (XRPD) gekennzeichnet ist: 5,8°±0,1°, 7,4°±0,1°, 16,0°±0,1°, 20,2°±0,1°, 23,5°±0,1°, 25,2°±0,1°.

4. Orale Darreichungsform nach einem der Ansprüche 1 bis 3, wobei in der Apixaban-Partikelgrößenverteilung das Verhältnis von D90-Wert zu D50-Wert zwischen 9:1 und 2:1 liegt.

5. Orale Darreichungsform nach einem der Ansprüche 1 bis 4, wobei das Gewichtsverhältnis von Apixaban zu Matrixbildner 1:1 bis 1:50 beträgt.

6. Orale Darreichungsform nach einem der Ansprüche 1 bis 5, wobei der Matrixbildner ein nicht erodierbares Polymer ist, wobei die nicht erodierbaren Polymere Cellulosederivate sind, wie Celluloseether und Celluloseester, Stärke, Maisstärke, Gummi, Schellack, synthetische Polymere wie Polyvinylchlorid, Polyvinylalkohol oder Derivate davon, Polyvinylacetat, Polyvinylacetat/Polyvinylpyrollidon (Kollidon), Polymere auf Basis von Acrylsäure und/oder Methacrylsäure und deren Derivaten, Ionenaustauscherharze wie Polacrilinkalium und deren Mischung.

7. Orale Darreichungsform nach einem der Ansprüche 1 bis 6, wobei der Matrixbildner eine Wasserlöslichkeit bei 25°C von weniger als 33 mg/l aufweist, bestimmt durch das Säulenelutionsverfahren gemäß der EU-Richtlinie 67/548 EWG, Anhang V, Kap. A6.

8. Orale Darreichungsform nach einem der Ansprüche 1 bis 7, wobei der Matrixbildner eine Substanz mit einem Quellverhältnis von 1,5 bis 4,5 aufweist, bestimmt nach Ph.Eur., 6.1, Kapitel 2.8.4.

9. Orale Darreichungsform nach einem der Ansprüche 1 bis 8, ferner umfassend einen (c1)-Füller.

10. Orale Darreichungsform nach Anspruch 9, wobei der Matrixbildner (b) und der Füllstoff (c1) in einer agglomerierten Mischung vorhanden sind.

11. Orale Darreichungsform nach einem der Ansprüche 1 bis 10, umfassend
2,5-20 mg Apixaban
20-100 mg Matrixbildner
20-100 mg Füllstoff
1-5 mg Schmiermittel und
1-5 mg Gleitmittel

12. Orale Darreichungsform nach einem der Ansprüche 1 bis 11 in Form einer Tablette mit einer Inhaltsgleichmäßigkeit von 95 bis 105%, einer Brüchigkeit von weniger als 5% und/oder einer Härte von 30 bis 180 N.

13. Orale Darreichungsform nach einem der Ansprüche 1 bis 12, wobei das Auflösungsprofil der Darreichungsform eine wesentliche kinetische Nullordnung im Bereich von 0 bis 80% Auflösung aufweist.

14. Verfahren zur Herstellung einer oralen Darreichungsform nach einem der Ansprüche 1 bis 13, umfassend die Schritte von
i) Mischen von Apixaban, Matrixbildner und gegebenenfalls weiteren pharmazeutischen Hilfsstoffen
ii) gegebenenfalls Granulieren der Mischung aus Schritt i)
iii) Verarbeiten der Mischung aus Schritt i) oder den Granulaten aus Schritt ii) und gegebenenfalls weiteren Hilfsstoffen zu einer oralen Darreichungsform.
iv) gegebenenfalls Beschichten der Darreichungsform mit einem Film.

## Revendications

1. Forme posologique orale à libération modifiée contenant
a) de l'apixaban particulaire et cristallin et
b) un agent générateur de matrice,
la distribution granulométrique de l'apixaban ayant une valeur D50 de 11 à 75 µm, et
l'agent générateur de matrice étant choisi parmi l'éther de cellulose, l'ester de cellulose, l'amidon, la gomme, la gomme, la gomme laque, les matières grasses, le chlorure de polyvinyle, l'acétate de polyvinyle et l'alcool polyvinylique, les polymères à base d'acide acrylique et/ou méthacrylique et les résines échangeuses d'ions, les matières grasses étant la cire, la graisse, l'huile, l'acide gras, l'alcool gras, le monoglycéride, le diglycéride, le triglycéride et leurs mélanges, et
la libération modifiée signifiant qu'après 60 minutes, 1 % à 20 % de l'agent actif sont libérés, déterminés selon le procédé USP, appareil à palette II, milieu d'essai de 900 ml, tampon phosphate avec 0,5 % de dodécyl sulfate de sodium, pH 6,8 à 37°C et 75 tr/min.

2. Forme posologique orale selon la revendication 1, la forme posologique présentant une libération de 1 % à 20 % après 60 minutes, de 15 % à 55 % après 4 heures et de 55 % à 95 % après 10 heures, déterminée selon le procédé USP, dispositif à palette II, milieu de test de 900 ml, tampon phosphate avec 0,5 % de dodécyl sulfate de sodium, pH 6,8 à 37°C et 75 tr/min.

3. Forme posologique orale selon la revendication 1 ou 2, l'apixaban étant présent sous la forme du polymorphe N1 ou sous la forme du polymorphe H2-2,
le polymorphe N1 étant **caractérisé par** les positions angulaires à deux thêta dans la diffraction des rayons X sur poudre (XRPD) : 10,0°±0,1°, 10,6°±0,1°, 12,3°±0,1°, 12,9°±0,1°, 18,5°±0,1°, 27,1°±0,1°, et
le polymorphe H2-2 étant **caractérisé par** les positions angulaires à deux thêta dans la diffraction des rayons X sur poudre (XRPD) : 5,8°±0,1°, 7,4°±0,1°, 16,0°±0,1°, 20,2°±0,1°, 23,5°±0,1°, 25,2°±0,1°.

4. Forme posologique orale selon l'une quelconque des revendications 1 à 3, dans laquelle, dans la distribution granulométrique de l'apixaban, le rapport de la valeur D90 à la valeur D50 est compris entre 9:1 et 2:1

5. Forme posologique orale selon l'une quelconque des revendications 1 à 4, le rapport pondéral de l'apixaban à l'agent générateur de matrice étant de 1 :1 à 1 :50.

6. Forme posologique orale selon l'une quelconque des revendications 1 à 5, l'agent générateur de matrice étant un polymère non érodable, les polymères non érodables étant des dérivés de cellulose, tels que l'éther de cellulose et l'ester de cellulose, l'amidon, l'amidon de maïs, la gomme, la gomme laque, les polymères synthétiques tels que le chlorure de polyvinyle, l'alcool polyvinylique ou ses dérivés, l'acétate de polyvinyle, l'acétate de polyvinyle/polyvinylpyrollidone (Kollidon), les polymères à base d'acide acrylique et/ou d'acide méthacrylique et leurs dérivés, les résines échangeuses d'ions comme la polacriline potassium, et leurs mélanges.

7. Forme posologique orale selon l'une quelconque des revendications 1 à 6, l'agent générateur de matrice ayant une solubilité dans l'eau à 25 °C inférieure à 33 mg/l, déterminée par le procédé d'élution sur colonne conformément à la directive européenne 67/548 CEE, annexe V, chapitre A6.

8. Forme posologique orale selon l'une quelconque des revendications 1 à 7, l'agent générateur de matrice comprenant une substance ayant un rapport de gonflement de 1,5 à 4,5, déterminé selon Ph.Eur., 6.1, chapitre 2.8.4.

9. Forme posologique orale selon l'une quelconque des revendications 1 à 8, comprenant en outre une charge (c1).

10. Forme posologique orale selon la revendication 9, l'agent générateur de matrice (b) et la charge (c1) étant présents dans un mélange aggloméré.

11. Forme posologique orale selon l'une quelconque des revendications 1 à 10 comprenant
2,5-20 mg d'apixaban
20-100 mg d'agent générateur de matrice
20-100 mg de matière de charge
1-5 mg de glissant, et
1-5 mg de lubrifiant.

12. Forme posologique orale selon l'une quelconque des revendications 1 à 11 sous la forme d'un comprimé ayant une uniformité de contenu de 95 à 105 %, une friabilité inférieure à 5 % et/ou une dureté de 30 à 180 N.

13. Forme posologique orale selon l'une quelconque des revendications 1 à 12, le profil de dissolution de ladite forme posologique présentant une cinétique substantielle d'ordre zéro dans la gamme de 0 à 80% de dissolution.

14. Procédé de préparation d'une forme posologique orale selon l'une quelconque des revendications 1 à 13 comprenant les étapes consistant à
i) mélanger de l'apixaban, un agent générateur de matrice et éventuellement d'autre(s) excipient(s) pharmaceutique(s)
ii) éventuellement granulation du mélange de l'étape i)
iii) transformation du mélange de l'étape i) ou des granulés de l'étape ii) et éventuellement d'autres excipients en une forme posologique orale
iv) en option l'enrobage en film de la forme posologique.
